(19) Europäisches Patentamt
European Patent Office
Office européen des brevets

(11) Veröffentlichungsnummer: **0 017 151**
**B1**

(12) # EUROPÄISCHE PATENTSCHRIFT

(45) Veröffentlichungstag der Patentschrift: **15.02.84**

(51) Int. Cl.³: **A 61 K 6/08,** C 07 C 147/02

(21) Anmeldenummer: **80101587.6**

(22) Anmeldetag: **26.03.80**

(54) **Kalthärtende Materialien für Dentalzwecke mit polymerisationsaktiven Sulfonen.**

(30) Priorität: **03.04.79 DE 2913220**

(43) Veröffentlichungstag der Anmeldung: **15.10.80 Patentblatt 80/21**

(45) Bekanntmachung des Hinweises auf die Patenterteilung: **15.02.84 Patentblatt 84/7**

(84) Benannte Vertragsstaaten: **DE**

(56) Entgegenhaltungen:
**DE - B - 1 026 526**
**DE - B - 1 030 561**
**FR - A - 1 386 369**
**FR - A - 2 206 930**
**US - A - 2 558 139**
**US - A - 2 758 106**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Schmitz-Josten, Robert, Dr., Gerstenkamp 6, D-5000 Koeln 80 (DE)**
Erfinder: **Walkowiak, Michael, Dr., Albertus-Magnus-Strasse 10, D-5090 Leverkusen 1 (DE)**
Erfinder: **Schulz, Hans-Hermann, Am Neulandkreuz 23, D-5653 Leichlingen 1 (DE)**
Erfinder: **Bömer, Bruno, Dr., Gustav-Freytag-Strasse 2, D-5090 Leverkusen 1 (DE)**
Erfinder: **Süling, Carlhans, Dr., Carl-Leverkus-Strasse 10, D-5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Kalthärtende Materialien für Dentalzwecke mit polymerisationsaktiven Sulfonen

Gegenstand der Erfindung ist ein kalthärtendes Material mit guter Aushärtung und Durchführung auf Gips, das für Dentalzwecke verwendet werden soll.

Es ist bekannt, mit Hilfe der Redox-Polymerisation bei Raumtemperatur die verschiedensten polymerisierbaren ungesättigten Verbindungen (Monomeren) in den polymeren Zustand zu überführen. Solche Verfahren sind beispielsweise beschrieben in der DE-PS 919 668 und bei W. Kern, Makromolekulare Chemie 1 (1948), S. 209 ff. Hierbei werden vorzugsweise Kombinationen aus einem Oxydationsmittel und einem Reduktionsmittel eingesetzt, wie zum Beispiel Benzoylperoxid in Kombination mit Benzolsulfinsäure.

Ferner ist bekannt, solche Redoxpolymerisationen von Monomeren in Gegenwart von Polymeren durchzuführen. In der Dentaltechnik werden beispielsweise giessfähige bis pastenförmige polymerisierbare Mischungen aus Methacrylsäuremethylester und Perlpolymerisaten, welche Methacrylsäuremethylester einpolymerisiert enthalten, zur Herstellung von Zahnprothesen, künstlichen Zähnen, Zahnfüllungen und Zahnreparaturmaterialien eingesetzt. Hierbei wird das Oxydationsmittel, zum Beispiel ein Peroxid, meist vor der Verwendung in Gegenwart des Perlpolymerisats gelagert, während das Reduktionsmittel im Monomeren gelöst wird. Beim Vermischen von Perlpolymerisat und Monomer werden die Redoxaktivatoren vereinigt und damit die Polymerisation ausgelöst. Solche Verfahren sind beispielsweise in der SZ-PS 233 040, FR-PS 876 279, FR-PS 890 289 und der DE-PS 973 590 beschrieben.

Anstelle/beziehungsweise in Kombination mit Perlpolymerisaten können auch anorganische Füllstoffe eingesetzt werden, wie die US-PS 3 066 112 und 3 926 906 lehren.

Häufig wird als Redox-Aktivierungssystem eine Kombination aus einem Peroxid und einem tertiären Amin verwendet, wie beispielsweise Benzoylperoxid und N,N-Dimethyl- beziehungsweise N,N-Bishydroxyethyl-p-toluidin, doch sind die dabei erhaltenen Polymerisationsprodukte wenig farbstabil; es gelingt auch bei Zusatz von UV-Stabilisatoren nicht, die bei der Wärmealterung auftretenden Verfärbungen zu unterdrükken. Es sind zahlreiche Versuche bekannt geworden, diesen Mangel zu beseitigen. So sollen nach der Lehre der DDR-PS 21 986 die 3,4-dialkylierten N,N-Dimethylaniline weniger starke Verfärbungen bewirken als das N,N-Dimethyl-p-toluidin (DMT). R. L. Bowen und H. Argentar (Journal of the American Dentist's Association 75 (1967), 918 bis 923) fanden, dass das N,N-Dimethyl-3,5-dimethylanilin weniger stark verfärbt als DMT.

Die US-PS 2 558 139 beschreibt weitere Redoxsysteme, die unter Verwendung von oxyalkylierten tertiären Aminen, wie Phenyl-diethanolamin, m-Tolyl-diethanolamin oder Triethanolamin als Cokatalysatoren aufgebaut sind. Das oxyethylierte p-Toluidin, die Oxyalkylierungsprodukte des 3,5- und 3,4-Dimethylanilins und des 3,5-Di-t-butylanilins sind in einer Reihe von Patentschriften als Cokatalysatoren für die Polymerisation spezieller Kompositionen für dentale Anwendungen beschrieben worden, so zum Beispiel in den DE-PS 1 020 183, US-PS 3 593 533, US-PS 3 541 068, US-PS 3 799 985, DE-PS 2 559 213 und DE-PS 2 658 538.

In der vorerwähnten US-PS 2 558 139 werden auch eine Reihe anderer Stickstoff und Schwefel enthaltender Verbindungsklassen erwähnt, welche als Cokatalysatoren geeignet sind, zum Beispiel Pyrazolone und Sulfimide, wie o-Benzoesäuresulfimid (Saccharin). Das Saccharin wird auch in anderen Veröffentlichungen als Cokatalysator in Kombination mit tertiären Aminen verwendet. So beschreibt die US-PS 2 833 735 die Verwendung der Saccharinsalze von tertiären aromatischen Aminen, die US 3 046 262 die Kombination von Saccharin mit Peroxiden und tertiären aliphatischen Aminen.

Weitere Modifikationen des Redox-Systems Peroxid-aromatisches Amin betreffen den Zusatz von Verbindungen des 2- oder 4wertigen Schwefels, wie Mercaptane (US-PS 2 744 886), Schwefeldioxid (DE-PS 956 810), Sulfinsäuren beziehungsweise sulfinsauren Salzen (DE-PS 927 052, DE-PS 1 006 616, DE-PS 2 739 282).

Eine weitere Gruppe von Schwefel und Aminstickstoff enthaltenden Polymerisationskatalysatoren sind die in den DE-PS 913 477, DE-PS 955 633, US-PS 2 750 357, US-PS 2 779 751 und US-PS 2 776 952 beschriebenen tertiären Amine, welche in α-Stellung zum Stickstoff eine Alkyl- oder Arylsulfongruppe enthalten, doch ist die Lagerfähigkeit der Monomeren in Gegenwart solcher Aminosulfone zeitlich begrenzt.

Eine andere Gruppe polymerisationsaktiver Schwefelverbindungen ist in der DE-PS 1 026 526 und DE-PS 1 030 561 beschrieben worden. Es handelt sich hierbei um Thioether oder Sulfone, welche in β-Stellung zum Schwefelatom ein Wasserstoffatom besitzen, und deren Aktivität als Polymerisationskatalysatoren durch Zugabe von basisch wirkenden Verbindungen, wie Aminen, quartäre Ammoniumbasen, Sulfoniumbasen oder Salzen von Alkali- oder Erdalkalimetallen mit schwachen Säuren erhöht werden kann. Viele der in der DE-PS 1 026 526 beschriebenen Sulfone sind jedoch weder in Gegenwart von Peroxiden noch in Gegenwart von basisch wirkenden Verbindungen hinreichend lagerfähig, was ihre praktische Verwertbarkeit in Zweikomponentensystemen beeinträchtigt.

Aufgabe der vorliegenden Erfindung ist es, ein kalthärtendes Material für Dentalzwecke zur Verfügung zu stellen, welches die erwähnte Verfärbung bei der Wärmealterung nicht zeigt und ausserdem auf feuchtem Gips gut aushärtet. Diese Aufgabe wird erfindungsgemäss durch ein Material auf Basis einer Mischung von Polymeren, Monomeren und Polymerisationskatalysatorsystemen gelöst, welches dadurch gekennzeichnet ist, dass das Polymerisationskatalysatorsystem eine Kombination aus

a) einem Peroxid,

b) einem aromatischen tertiären Amin,

c) einem Sulfon, welches in β-Stellung zur Sulfongruppe ein aktiviertes Wasserstoffatom besitzt, und

d) eine zur Aktivierung der Komponente c) geeignete basisch wirkende Verbindung umfasst.

Die erfindungsgemäss zu verwendende Katalysatorkombination aus einem konventionellen Redox-Initiatorsystem (aromatisches tertiäres Amin/Peroxid) und dem bekannten System Sulfon mit aktiviertem Wasserstoff in β-Stellung/aktivierend wirkende basische Verbindung wird durch den Stand der Technik nicht vorweggenommen oder auch nur nahegelegt. Insbesondere war nicht zu erwarten, dass derartige Kombinationen frei sind von den Nachteilen der einzelnen Katalysatorsysteme.

Als Peroxide werden erfindungsgemäss vorzugsweise Diacylperoxide, wie Benzoylperoxid, p-Chlorbenzoylperoxid oder Lauroylperoxid verwendet. Besonders bevorzugt wird das o-Toluylperoxid eingesetzt.

Als aromatische Amine können verwendet werden: N,N-Dimethyl-anilin,
N,N-Dimethyl-toluidin,
N,N-Dimethyl-3,4-dimethylanilin,
N,N-Dimethyl-3,5-dimethylanilin,
N,N-Dimethyl-3,4,5-trimethylanilin;
vorzugsweise werden jedoch aromatische tertiäre Amine verwendet, welche in der aliphatischen Seitenkette mindestens eine Hydroxylgruppe tragen, wie
N-Methyl-N-hydroxylpropyl-3,5-dimethylanilin,
N,N-Bis-(hydroxyethyl)-p-toluidin,
n,N-Bis-(hydroxypropyl)-p-toluidin
N,N-Bis-(hydroxyethyl)-3,4-dimethylanilin
N,N-Bis-(Hydroxypropyl)-3,4-dimethylanilin,
N,N-Bis-(hydroxyethyl)-3,5-dimethylanilin
N,N-Bis-(hydroxypropyl)-3,5-dimethylanilin
N,N-Bis-(hydroxyethyl)-3,5-di-t-butylanilin
N,N-Bis-(hydroxyethyl)-4-t-butylanilin.

Geeignete Sulfone mit aktivierten β-ständigen Wasserstoffatomen sind in der DE-PS 1 026 526 beschrieben. Bevorzugt sind die Sulfone der Formel I

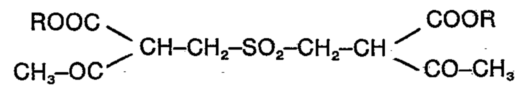

I,

wobei R eine Methyl- oder Ethylgruppe bedeuten kann. Sie zeichnen sich durch eine besonders gute Lagerfähigkeit in Gegenwart von Peroxiden und zusätzlich eine starke Reduktionswirkung in Gegenwart von basisch wirkenden Verbindungen aus. Die Herstellung von Verbindungen der Formel I kann nach einem Ein- oder Zweistufenverfahren gemäss nachstehendem Formelschema aus Derivaten der Sulfoxylsäure, wie Natriumhydrosulfit oder hydroxymethansulfinsaurem Natrium, geschehen:

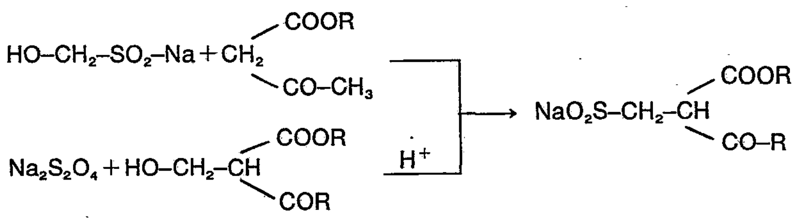


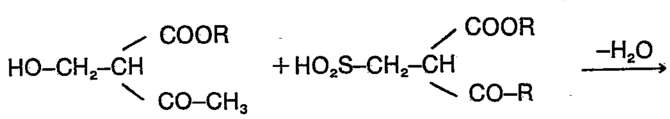


I

Die zur Aktivierung der obengenannten Sulfone geeigneten basisch wirkenden Verbindungen sind dem Fachmann bekannt (die Basizität tertiärer aromatischer Amine reicht zur Aktivierung der Sulfone bekanntlich nicht aus). Als Komponente d) können erfindungsgemäss beispielsweise verwendet werden primäre, sekundäre oder tertiäre aliphatische oder araliphatische Amine, wie Dibutylamin, N-t-Butylaminoethyl-methacrylat, N,N-Dimethylbenzylamin, N,N-Dimethylamino-ethylmethacrylat sowie die Salze von Oniumverbindungen des Stickstoffs, Schwefels oder Phosphors mit schwachen Säuren, wie Methacrylsäure oder Essigsäure.

Vorzugsweise werden als basisch wirkende Verbindungen quartäre Ammoniumsalze von Sulfimiden verwendet, z.B. quartären Ammoniumsalzen des o-Benzoesäuresulfimids (Saccharin), wobei die quartären Ammoniumbasen mindestens eine Alkylgruppe mit mehr als neun Kohlenstoffatomen enthalten sollen, wie z.B.
N,N-Dimethyl-n-decyl-benzylammonium-saccharinat.

Es können auch Gemische der Salze von quartären Ammoniumbasen mit Saccharin verwendet werden. Besonders geeignet ist das leicht zugängliche Umsetzungsprodukt aus je einem Mol Alkalisaccharinat und einem Gemisch von ca. 70% n-Dodecyl- und ca. 30%
Tetradecyl-di-methyl-benzylammoniumchlorid.

Das letzere Gemisch befindet sich unter dem Warenzeichen Zephirol® der Bayer AG, Leverkusen auf dem Markt. Das Saccharinat kann leicht durch Umsetzung der Komponenten in wässriger

Lösung dargestellt werden, da es in Wasser schwer löslich ist. Solche hydrophoben quartären Ammoniumsalze haben den Vorteil, dass bei ihrer Verwendung die durch Wasseraufnahme aus den verwendeten Gipsformen leicht eintretende Weissverfärbung der Dentalkunststoffe vermindert wird.

Eine bevorzugte Ausführungsform der vorliegenden Erfindung besteht darin, dass man das Peroxid a) und das Sulfon c) zusammen, gegebenenfalls in Gegenwart eines inerten Verdünnungsmittels, z.B. Perlpolymerisaten von Methacrylsäuremethylester, oder anorganischen Füllstoffen wie silanisiertem Quarz, oder auch in einem Weichmacher, wie Phthalsäuredibutylester oder Phthalsäurediallylester lagert, während das aromatische tertiäre Amin b) und die zur Aktivierung des Sulfons verwendete Verbindung d) in dem zu polymerisierenden Monomeren gelagert werden.

Bei einer anderen Ausführungsform werden das Peroxid a) und das Sulfon c) einerseits sowie das aromatische tertiäre Amin b) zusammen mit der das Sulfon aktivierenden basisch wirkenden Verbindung anderseits mit den zu polymerisierenden Monomeren getrennt vermischt und gelagert, wobei Füllstoffe, UV-Schutzmittel oder Pigmente einer oder beiden Mischungen zugesetzt werden können. Die Polymerisation tritt beim Vermischen der getrennt gelagerten Komponenten bei Raumtemperatur in kurzer Zeit ein.

Die einzusetzenden Mengen der Komponenten a) bis d) betragen in Gew.-% (bezogen auf das zu polymerisierende Monomere) 0,1 bis 4% Peroxid, 0,1 bis 2 aromatisches Amin, 0,05 bis 1,5% basisch wirkende Verbindung und 0,1 bis 2% Sulfon. Eine bevorzugte Ausführungsform der Erfindung enthält jeweils 25 bis 50 Mol-% aromatisches tertiäres Amin, basisch wirkender Verbindung und Sulfon, bezogen auf das eingesetzte Peroxid.

Als polymerisierbare Monomere, welche mit dem erfindungsgemässen Polymerisationskatalysatorsystem zu Dental-Kunststoffen ausgehärtet werden können, werden vorzugsweise die Ester der Acrylsäure oder Methacrylsäure mit ein- oder mehrwertigen Alkoholen, welche noch weitere funktionelle Gruppen, wie Hydroxyl-, Alkoxy-, Epoxy-, Amino-, Allyl-, Isocyanato-, Urethan- oder Harnstoffgruppen enthalten können, verwendet. Sie können auch in Mischung miteinander eingesetzt werden. Beispielhaft seien als geeignete Monomere für das beanspruchte Verfahren genannt:

Methacrylsäure, Acrylsäure, Acrylamid, Methacrylamid, Methylmethacrylat, Ethylacrylat, Butylacrylat, Isobutylmethacrylat, 2-Ethylhexylmethacrylat, Cyclohexylmethacrylat, Cyclohexyl-dimethacrylat, Dodecylmethacrylat, Stearylmethacrylat, 2-Hydroxy-ethyl-acrylat, 2-Hydroxypropyl-methacrylat, 2-Butoxyethylmethacrylat, Methoxy-2-ethoxyethylmethacrylat, 2-Allyloxyethyl-methacrylat, Allylmethacrylat, Glycidylmethacrylat, Furfurylmethacrylat, 2-Dimethylaminoethyl-methacrylat, 2-t-Butylaminoethylmethacrylat, 2-Bromoethyl-methacrylat, 2-Cyanoethyl-methacrylat, 1,1-Dihydroperfluorobutyl-methacrylat, Hexafluoroisopropyl-methacrylat, Phenylmethacrylat, 2,4,6-Tribromophenyl-methacrylat, Benzyl-methacrylat, Tribromoneopentyl-methacrylat, Bicyclo-(2,2,1)-5-hepten-2-methylmethacrylat, Ethylenglycoldiacrylat, Diethylenglycol- und Triethylenglycol-dimethacrylat, Polyethylenglycoldiacrylat, 1,3-Butandiol-diacrylat, 1,6-Hexandiol-dimethacrylat, Neopentylglycol-dimethacrylat, Trimethylolpropan-trimethacrylat, Pentaerythrit-tetraacrylat, Triacroylformal, Di-(hydroxymethyl)-tricyclo[5.2.1.$0^{2.6}$]-decylmethacrylat.

Beispiele weiterer geeigneter Monomere sind in der nachstehenden Zusammenstellung formelmässig aufgeführt. In diesen Formeln bedeuten

$R = CH_2 = C{-}CO{-}$ oder $CH_2 = CH{-}CO{-}$,
$\quad\quad\quad\quad |$
$\quad\quad\quad\quad CH_3$

$R_1 = H$ oder $-CH_2-OR$,

$n = 1–4$ und

$m = 0–4$,

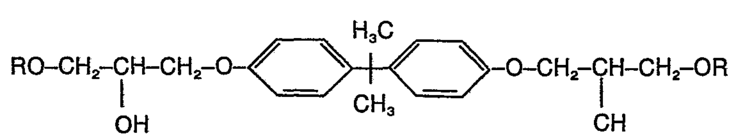

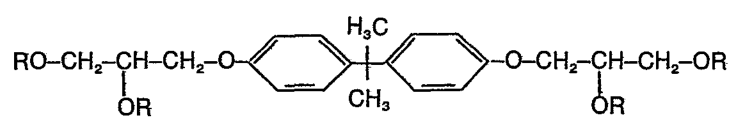

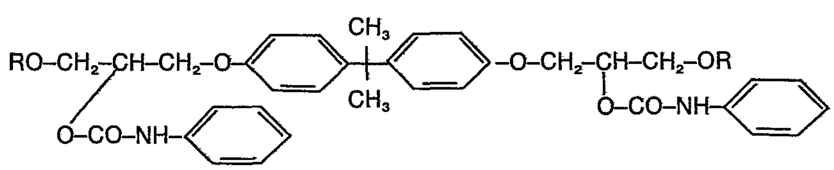

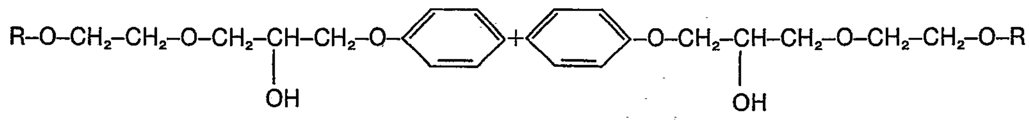

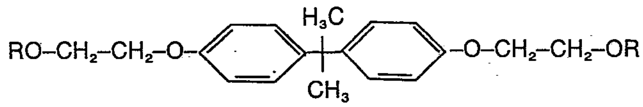

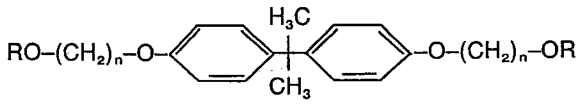

n = >2

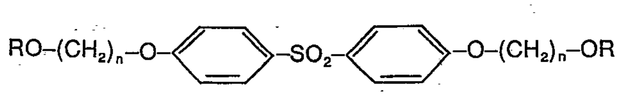

n = 2–4

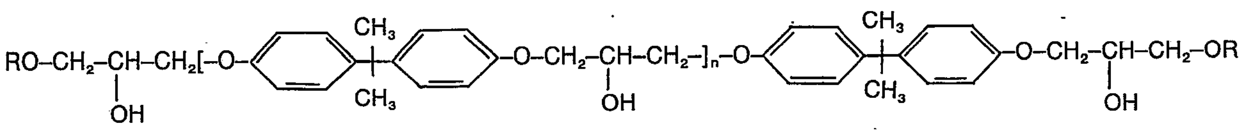

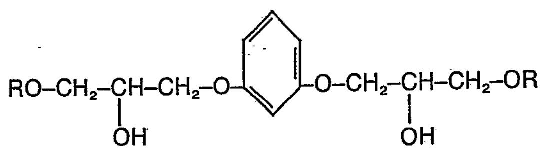

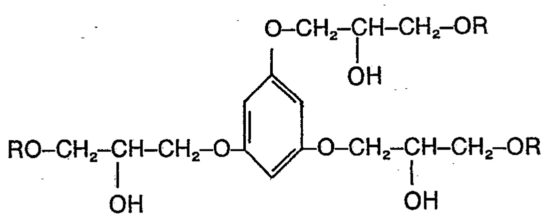

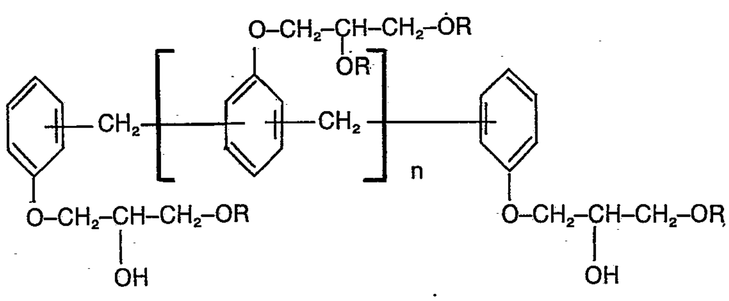

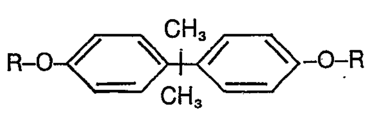

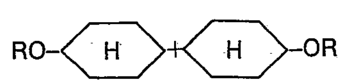

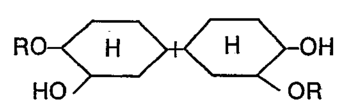

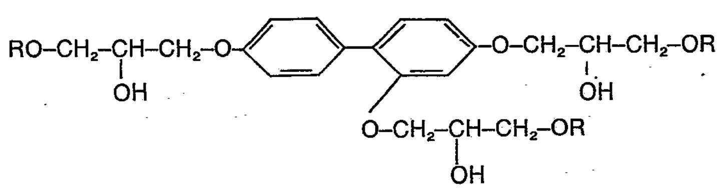

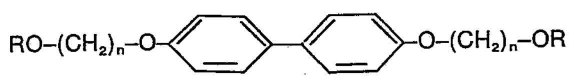

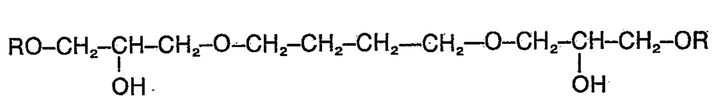

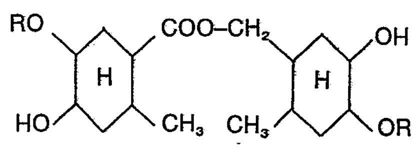

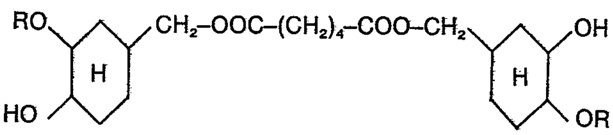

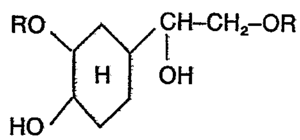

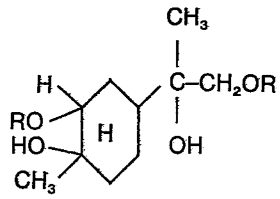

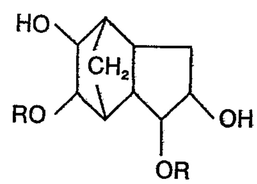

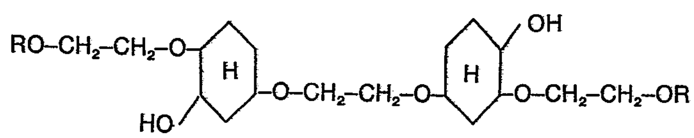

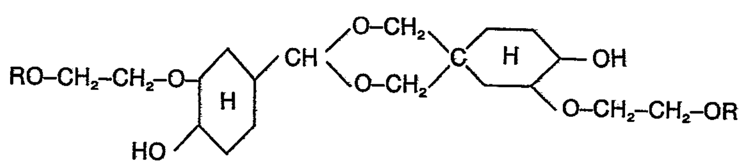

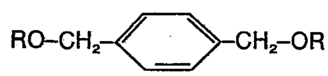

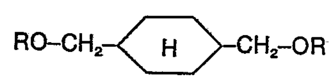

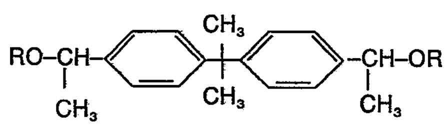

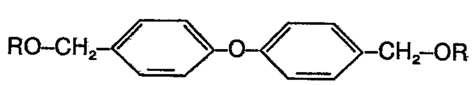

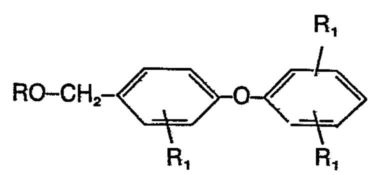

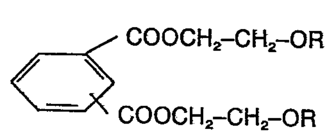

in der ortho-, meta- oder para-Form

Verbindung der allgemeinen Formel: $R-(O-D-O-X)_n-OD-OR$, wobei HO–D–OH ein Polyol und HO–X–OH eine Dicarbonsäure ist, und zwar jeweils gesättigt oder ungesättigt, cyclisch oder acyclisch.

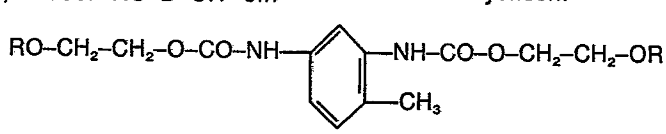

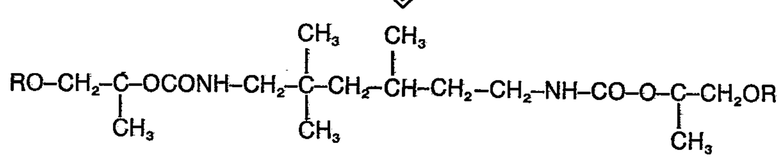

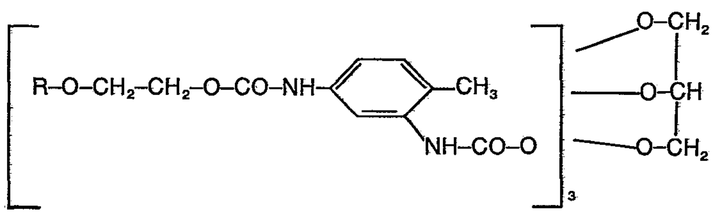

(Durchschnitts-Molgewicht 2500)

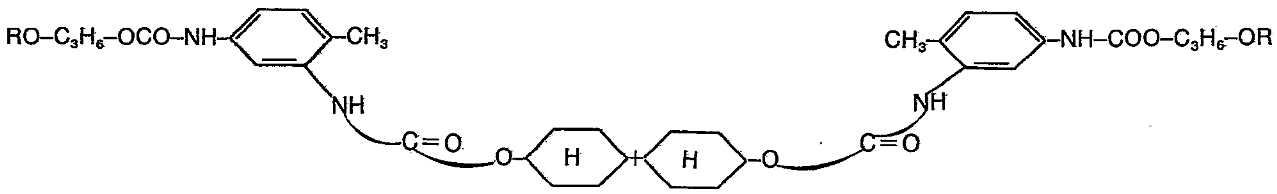

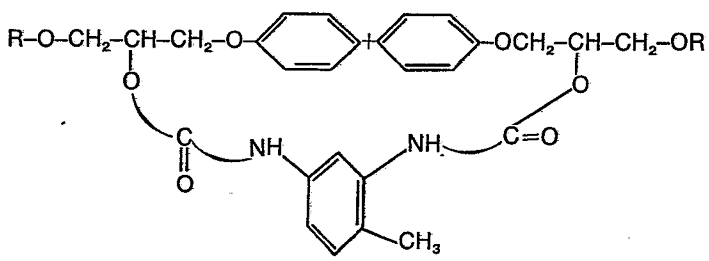

Ferner eignen sich NCO- und Urethangruppen enthaltende Prepolymere, umgesetzt mit Hydroxyalkyl-methacrylat gemäss DT-OS 2 419 887 oder FR-PS 2 271 807.

Die erfindungsgemässen Materialien können verwendet werden als Press-, Giess- und Streichmassen. Sie werden mit Vorteil auf dem Gebiet der Dentaltechnik verwendet zur Herstellung von Prothesenmaterialien, Zahnreparaturmassen, Brücken- und Kronenmaterialien und Verblendmassen, Überzugs- und Versiegelungsmassen, Grundiermaterialien für Kavitäten usw.

Beispiel 1

Herstellung von Bis-(methylen-acetessigsäuremethylester-)sulfon (Formel I, $R = CH_3$).

Man kühlt eine Mischung aus 800 g Methanol, 412 g Acetessigsäuremethylester und 380 g einer 30%igen wässrigen Formaldehydlösung auf −30 °C und tropft unter Rühren innerhalb von 30 Minuten eine Lösung von 2,8 g Kaliumcarbonat in 28 g Wasser hinzu, wobei sich ein pH-Wert von 9 einstellt. Man rührt noch 30 Minuten bei −10 °C nach und fügt dann 84 g Eisessig hinzu. Diese auf −10 °C gekühlte Lösung wird dann zu einer Lösung aus 145 g Natriumdithionit, 300 g Wasser und 700 g Eis gegossen und eine Stunde bei −6 bis −10 °C gerührt. Anschliessend tropft man innerhalb einer Stunde 100 ml einer 10normalen Schwefelsäure zu und rührt den Ansatz 16 Stunden bei 0–5 °C.

Die gebildeten Kristalle werden abgesaugt und mit Wasser gewaschen. Anschliessend werden sie mit 600 ml Wasser und 12 ml 35%igem Wasserstoffperoxid drei Stunden verrührt, abgesaugt und mit Methanol gründlich ausgewaschen.

Man erhält 150–230 g des Sulfons vom Schmelzpunkt 146–149 °C, welches aus viel Dioxan umkristallisiert wird. Fp. 151 °C.

Beispiel 2

Herstellung eines Saccharinates von quartären Ammoniumbasen.

43 g Benzoesäure-2-sulfonimid (Saccharin) und 13 g Natriumcarbonat werden in 1000 ml Wasser gelöst, wobei ein pH-Wert von 7–8 eingestellt wird. Innerhalb von 2 Stunden werden 150 g einer 50%igen wässrigen Lösung von ca. 70% n-Dodecyl- und ca. 30% Tetradecyl-dimethyl-benzylammoniumchlorid unter Rühren zugetropft und 0,5 Stunden nachgerührt. Das Produkt wird abgesaugt, zweimal mit je 200 ml Wasser gründlich ausgewaschen und bei 40 °C i.V. getrocknet.

Ausbeute 105; Schmp. 90–92 °C.

Beispiel 3

1. 940 g Methacrylsäuremethylester, 60 g entstabilisiertes Ethylenglycoldimethacrylat, 5 g 2-(2′-Hydroxy-5′-methylphenyl)-benzotriazol, 10 g Saccharinat nach Beispiel 2, 4 g N,N-Bis-2-hydroxypropyl-3,5-dimethylanilin (Stereoisomerengemisch), 2,15 g Methacrylsäure und 2,0 g Tetrabutylammoniumbromid werden homogen vermischt.

2. Ein Perlpolymerisat-Gemisch, bestehend aus

a) 90 Gew.-% Copolymer aus 95% Methylmethacrylat und 5% Acrylsäureethylester, $L_z = 55\ \mu$, und

b) 10 Gew.-% Copolymer aus 75% Methylmethacrylat und 25% Acrylsäureethylester, $L_z = 110\ \mu$, wird mit 1,1% feinteiligem o-Toluylperoxid und 0,65%

Bis-(methylenacetessigsäuremethylester)-sulfon gemäss Beispiel 1 vermischt und durch Sieben homogenisiert.

5 Teile Pulver 1) und 3 Teile Flüssigkeit 2) werden 30 Sekunden lang innig vermischt und in eine mit Natriumalginat isolierte Gipsform gegossen. Die verschlossene Form wird in einem Druckbehälter 1 Stunde unter einem Druck von 2 bar sich selbst überlassen. Beim Entformen wird ein farbloser Formkörper erhalten, der eine glatte Oberfläche aufweist.

Der erhaltene Formkörper hat folgende Festigkeitseigenschaften: Schlagzähigkeit 19,6 kp/cm², Biegefestigkeit 1001 kp/cm², Biegewinkel 22,4°; Brinellhärte 1137/1011 bei 10″/60″. Bei einer Wärmealterung im Trockenschrank bei 60 °C wurde nach 2 Tagen keine Verfärbung der Proben festgestellt.

Werden die Versuche ohne das Sulfon gemäss Beispiel 1 durchgeführt, so verfärbt sich die Probe bei der Wärmealterung bei 60 °C.

Beispiel 4

Herstellung von Bis-(methylenacetessigsäureethylester)-sulfon (Formel I, $R=C_2H_5$).

Lösung 1

80 g hydroxymethansulfinsaures Natrium (Rongalit C®), 750 g Wasser und 65 g Acetessigsäureethylester werden unter Einleiten von Kohlendioxid 18 Stunden gerührt. Dabei tritt eine geringe Erwärmung auf. Nicht umgesetzter Acetessigester wird dann mit Petrolether ausgeschüttelt.

Lösung 2

65 g Acetessigsäureethylester und 55 g wässrige 30%ige Formaldehyd-Lösung werden auf −20 °C gekühlt. Man tropft nun 5 g einer 10%igen Lösung von Kaliumcarbonat in Wasser zu und hält das Reaktionsgemisch 0,5 bis 1 Stunde auf −15 °C bis −20 °C. Dann tropft man diese Lösung zur auf −10 °C gekühlten und mit 100 g Eis versetzten Lösung I zu und versetzt das Gemisch sofort mit 50 ml einer 10normalen Schwefelsäure und mit 100 ml Petrolether. Man rührt drei Tage unter Eis-Kühlung und saugt die gebildeten Kristalle ab. Diese werden in Essigsäureethylester gelöst, vom Ungelösten abfiltriert und im Vakuum bei 30 °C eingeengt. Nach Zusatz von Ethanol und Impfkristallen unter Kühlung kristallisiert das Sulfon aus. Es wird mit eiskaltem Ethanol gewaschen und getrocknet.

Schmelzpunkt: 88 °C; Schwefelanalyse: 9,35% (theor. 9,15%).

## Patentansprüche

1. Kalthärtendes Material für Dentalzwecke, enthaltend eine Mischung von Polymeren, polymerisierbaren Monomeren und einem Polymerisationskatalysatorsystem, dadurch gekennzeichnet, dass das Polymerisationskatalysatorsystem eine Kombination aus

a) einem Peroxid,

b) einem aromatischen tertiären Amin,

c) einem Sulfon, welches in β-Stellung zur Sulfongruppe ein aktiviertes Wasserstoffatom besitzt, und

d) eine zur Aktivierung der Komponente c) geeignete basisch wirkende Verbindung umfasst.

2. Kalthärtendes Material nach Anspruch 1, dadurch gekennzeichnet, dass das Polymerisationskatalysatorsystem als Peroxid ein Diacylperoxid enthält.

3. Kalthärtendes Material nach Anspruch 1 oder 2, dadurch gekennzeichnet, dass das Polymerisationskatalysatorsystem als Peroxid o-Tolylperoxid enthält.

4. Kalthärtendes Material nach Anspruch 1 bis 3, dadurch gekennzeichnet, dass das Polymerisationskatalysatorsystem ein aromatisches, tertiäres Amin enthält, welches eine aliphatische Seitenkette mit mindestens einer Hydroxylgruppe trägt.

5. Kalthärtendes Material gemäss Anspruch 1 bis 4, dadurch gekennzeichnet, dass das Polymerisationskatalysatorsystem als Komponente d) Salze von Sulfimiden mit quartären Ammoniumbasen enthält.

6. Kalthärtendes Material gemäss Anspruch 5, dadurch gekennzeichnet, dass Komponente d) ein Salz des o-Benzoesäuresulfimids mit einer solchen quartären Ammoniumbase umfasst, die mindestens eine Alkylgruppe mit mehr als 9 Kohlenstoffatomen enthält.

7. Kalthärtendes Material gemäss Anspruch 1 bis 6, dadurch gekennzeichnet, dass das Polymerisationskatalysatorsystem als Komponente c) ein Sulfon der Formel

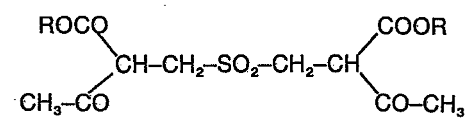

enthält, in welcher R für eine Methyl- oder Ethylgruppe steht.

8. Verfahren zur Herstellung eines Kunststoffs für dentale Anwendungszwecke durch Polymerisation eines Gemisches von polymeren und polymerisierbaren Monomeren in Gegenwart eines Redox-Polymerisationskatalysatorsystems auf Basis von Peroxid und aromatischem tertiärem Amin, dadurch gekennzeichnet, dass das Polymerisationskatalysatorsystem zusätzlich ein Sulfon, welches in β-Stellung zur Sulfongruppe ein aktiviertes Wasserstoffatom besitzt, sowie eine zur Aktivierung des Sulfons geeignete basisch wirkende Verbindung umfasst.

## Claims

1. A cold-curing material for dental purposes, containing a mixture of polymers, polymerisable monomers and a polymerisation catalyst system, characterised in that the polymerisation catalyst system comprises a combination of

a) a peroxide,

b) an aromatic tertiary amine,

c) a sulphone which has an activated hydrogen atom in the β-position relative to the sulphone group, and

d) a compound which has a basic action and is suitable for activating component c).

2. Cold-curing material according to Claim 1, characterised in that the polymerisation catalyst system contains a diacyl peroxide as the peroxide.

3. Cold-curing material according to Claim 1 or 2, characterised in that the polymerisation catalyst system contains o-tolyl peroxide as the peroxide.

4. Cold-curing material according to Claim 1 to 3, characterised in that the polymerisation catalyst system contains an aromatic, tertiary amine which carries an aliphatic side chain containing at least one hydroxyl group.

5. Cold-curing material according to Claim 1 to 4, characterised in that the polymerisation catalyst system contains salts of sulphimides with quaternary ammonium base as component d).

6. Cold-curing material according to Claim 5, characterised in that component d) comprises a salt of o-benzoic acid sulphimide with a quaternary ammonium base which contains at least one alkyl group with more than 9 carbon atoms.

7. Cold-curing material according to Claim 1 to 6, characterised in that the polymerisation catalyst system contains as component c) a sulphone of the formula

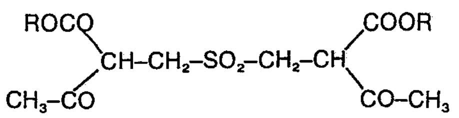

in which

R represents a methyl or ethyl group.

8. Process for the preparation of a plastic material for dental purposes of application by polymerising a mixture of polymers and polymerisable monomers in the presence of a redox polymerisation catalyst system based on a peroxide and an aromatic tertiary amine, characterised in that the polymerisation catalyst system additionally comprises a sulphone, which has an activated hydrogen atom in the β-position relative to the sulphone group, and a compound which has a basic action and is suitable for activating the sulphone.

**Revendications**

1. Matériau durcissant à froid pour applications dentaires, qui contient un mélange de polymères, de monomères polymérisables et d'un système catalyseur de polymérisation, caractérisé en ce que le système catalyseur de polymérisation comprend une combinaison de:

a) un peroxyde,

b) une amine aromatique tertiaire,

c) une sulfone portant un atome d'hydrogène activé en position β du groupe sulfone, et

d) un composé basique convenant pour l'activation du composant c).

2. Matériau durcissant à froid selon la revendication 1, caractérisé en ce que le système catalyseur de polymérisation contient en tant que peroxyde un peroxyde de diacyle.

3. Matériau durcissant à froid selon la revendication 1 ou 2, caractérisé en ce que le système catalyseur de polymérisation contient en tant que peroxyde de peroxyde d'o-tolyle.

4. Matériau durcissant à froid selon les revendications 1 à 3, caractérisé en ce que le système catalyseur de polymérisation contient une amine aromatique tertiaire qui porte une chaîne latérale aliphatique contenant au moins un groupe hydroxy.

5. Matériau durcissant à froid selon les revendications 1 à 4, caractérisé en ce que le système catalyseur de polymérisation contient en tant que composant d) des sels de sulfimide et de bases d'ammonium quaternaire.

6. Matériau durcissant à froid selon la revendication 5, caractérisé en ce que le composant d) comprend un sel de l'o-benzosulfimide et d'une base d'ammonium quaternaire contenant au moins un groupe alkyle à plus de 9 atomes de carbone.

7. Matériau durcissant à froid selon les revendications 1 à 6, caractérisé en ce que le système catalyseur de polymérisation contient en tant que composant c) une sulfone de formule

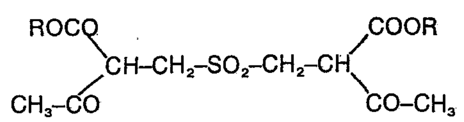

dans laquelle R représente un groupe méthyle ou éthyle.

8. Procédé de préparation d'une résine synthétique pour applications dentaires par polymérisation d'un mélange de polymère et d'un monomère polymérisable en présence d'un système catalyseur de polymérisation rédox à base d'un peroxyde et d'une amine aromatique tertiaire, caractérisé en ce que le système catalyseur de polymérisation contient en outre une sulfone portant un atome d'hydrogène activé en position β du groupe sulfone ainsi qu'un composé basique convenant pour l'activation de la sulfone.